# EUROPEAN PATENT APPLICATION

(11) **EP 2 949 657 A2**
(43) Date of publication of application: **02.12.2015**
(21) Application number: 14742870.0
(22) Date of filing: 23.01.2014
(51) Int. Cl.: C07K 7/06, C07K 14/655, A61K 38/08, A61K 38/31, A61K 51/08, A61K 103/00

(54) **CYCLIC OCTAPEPTIDE, RADIOPHARMACEUTICAL AGENT BASED THEREON AND METHOD FOR USING A RADIOPHARMACEUTICAL AGENT TO PRODUCE MEDICINAL (PHARMACEUTICAL) AGENTS FOR THE TREATMENT OF NEOPLASMS WHICH EXPRESS SOMATOSTATIN RECEPTORS**

(30) Priority: 25.01.2013 RU 2013103410
(71) Applicant: Zakrytoe Aktsionernoe Obschestvo "Pharm-Sintez", Moscow 111024 (RU)
(72) Inventor: NAZARENKO, Anna Borisovna, Moscow 121614 (RU); VOLOZNEV, Lev Vasilievich, Severouralsk Sverdlovskaya obl. 164482 (RU); GRININ, Maxim Gennadievich, Dmitrovograd Ulyanovskaya obl. 433504 (RU)
(74) Representative: Meissner, Bolte & Partner GbR
(86) International application number: PCT/RU2014/000050
(87) International publication number: WO 2014/116144

(57) **Abstract**

The invention refers to cyclic octapeptides being analogues of somatistatin, which can be applied for obtaining of various pharmaceutical means applied upon diagnostics or treatment, i.e. refers to the area of chemistry and medicine. Octapeptide meets the general claims (I): 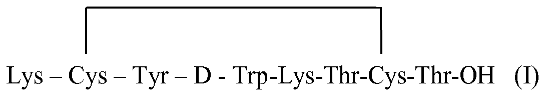 and it is intended for obtaining of radiopharmaceutical means with radionuclides ¹¹¹In, ⁹⁰Y, ¹⁷⁷Lu.

Cyclic octapeptide contains terminal amino acid lysine (Lys) capable of covalent attachment of the chelating agent and thus having the following structure (Ia) and (Ib) 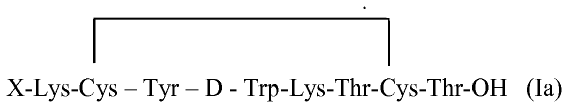 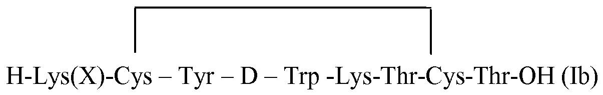 X = chelating agent

Cyclic octapeptide of the general claims (I) is a biologically active molecule, and being a somatostatin analogue, cyclic octapeptide under invention is applied for obtaining of radiopharmaceutical means with radionuclides ¹¹¹In, ⁹⁰Y, ¹⁷⁷Lu

The obtained radiopharmaceutical means can be applied for obtaining of the medical preparations and treatment of neoplasms (tumors) expressing somatistain receptors.

Long with that, compounds of the claim (I) with radionuclides ¹¹¹In, ⁹⁰Y, ¹⁷⁷Lu are obtained with the use of the chelating agents. These octapeptides under the invention inhibit release of such hormones as growth hormone, lactogenic hormone, decrease secretion of insulin, glucagon, thrombotonin; can be applied for the treatment of acromegaly, diabetes, acute pancreatitis.

3 independent claims, 1 dependent claim, 2 tables, 6 examples, 1 figure.

## Description

The invention belongs to the chemical and pharmaceutical industry and relates to pharmaceutical, radiopharmaceutical (hereinafter - RPP) preparations on the basis peptide carriers with the targeted delivery.

This invention belongs to peptide compounds, in particular to synthetic octapeptide regulating different biological processes and applied for obtaining of different medical agents, radiopharmaceutical preparations for diagnostics and treatment.

Peptides are a family of substances, whose molecules are built from the remaining α-amino acids combined into chain by peptide (amide) links.

Peptides are natural or synthetic compounds containing tenths, hundreds or thousands of monomer units - amino acids. Polypeptides consist of amino acids, oligopeptides consist of a small quantity of amino acids (no more than 10-50), and simple peptides contain up to 10 amino acids.

Peptides continuously synthesize in all living organisms for the regulation of physiological processes. Peptides properties mostly determine on their initial structure - sequence of amino acids, as well as on the molecule structure and its configuration in the space (secondary structure).

Peptides formation in the organism occurs within a few minutes, while chemical synthesis in the laboratory conditions is quite a long process, which can take several days, and synthesis technology development - several years. However, despite this, some weighty arguments in favor of works performance on the synthesis of natural peptides analogues exist.

At first, by means of the peptides chemical modification it is possible to confirm the primary structure hypothesis. Amino acid sequences of some hormones became known due to the synthesis of their analogues in the laboratory.

Secondly, synthetic peptides allow to study deeper the correlation between the structure of the amino acid sequence and its activity. For determination of the correlation between a certain peptide structure and its biological activity, the enormous work on synthesis of several thousands of analogues was performed. In the result, it was discovered that replacement of merely one amino acid in the peptide structure is able to increase it biological activity by several times or change its orientation. Along with that, change of the amino acid sequence length helps to determine location of the peptide active cores and the section of the receptor interaction.

Thirdly, thanks to the modification of the initial amino acid sequence, the opportunity to obtain pharmaceutical preparations appeared. Creation of the natural peptides' analogues allows detecting more "effective" configurations of molecules, which potentiate biological activity or make it longer lasting.

Fourthly, chemical synthesis of peptides is economically sound. Most pharmaceutical preparations would cost ten times more is they were produced on the basis of the natural product.

Active peptides are often discovered in nature only in nanogram quantities. Moreover, methods of peptides purification and extraction from the natural resources cannot completely divide the searched amino acid sequence with the peptides of the opposite or the same action. As for the specific peptides synthesized by the human organism - they can be obtained only by means of synthesis in the laboratory conditions.

A biologically active octapeptide Angiotensin II is known, which is formed from the large plasma protein of angiotensinogen in the result of two proteolytic enzymes action.

First proteolytic enzyme renin removes a peptide from the angiotensinogen N-terminal, which contains 10 amino acids and is called Angiotensin I. The second proteolytic enzyme carboxy-dipeptidyl peptidase removes 2 amino acids from the angiotensin C-terminal, in the result of which biologically active angiotensin II is formed, which participates in the regulation of the arterial pressure and water-salt metabolism in the organism.

Octapeptide Angiotensin II has the following amino acid sequence:

Asp - Arg - Val - Tyr - Ile - His - Pro - Phe

Functions of the peptides determine on their structure. Structure of angiotensin I is very similar to the one of angiotensin II (it has only two additional amino acids from the C-terminal), but at the same time it does not have any biological activity.

From the art another octapeptide is also known - octreotide (cyclic peptide) applied in pharmaceutical chemistry.

Octreotide is a synthetic analogue of somatostatin, which possesses similar profile of pharmaceutical activity, but is significantly superior over natural peptide in respect of its power and effect duration. Similarly to somatostatin, it inhibits secretion of somatotropin-releasing hormone in hypothalamus and secretion of the somatotrophic hormone and thyrotropin in the hypophysis anterior lobe, as well as suppresses secretion of various hormone active peptides (insulin, glucagon, gastrin, cholecystokinin, vasoactive and interstinal peptide, insulin-like growth factor-1) and thrombotonin produced by the gastrointestinal organs (stomach, bowel, liver and pancreatic gland).

Octreotide is applied as a medicinal agent for the treatment of acromegaly, tumors of gastro-pancreatic terminalocrine system, as well as used as an effective means of pancreatic surgery complications prevention.

Octreotide is represented as a cyclic octapeptide with the following structure:

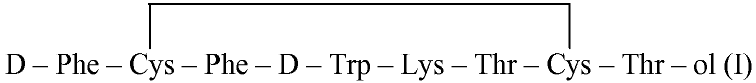

Peculiarities of its structure are as follows:
- Presence of two D-amino acids;
- Presence of the disulfide cycle;
- Recovered C-terminal residue of threonine (threoninol);
- High content of the hydrophobic aromatic amino acids.

Significant aspect from the point of chemical synthesis is the presence of tryptophane reside which is not resistant to oxidizers and strong acids effect. Octreotide synthesis can be performed both by the solid-phase method and by classicl methods of peptide synthesis in a solution.

Main challenges of octreotide synthesis by the solid-phase method are connected with the presence of threoninol C-terminal residue in its molecule. Threoninol does not contain carboxyl group, which does not provide an opportunity is apply traditional methods of the first amino acid (C-terminal) attachment to the polymer matrix. In the work by W.B. Edwards et al. (J. Med. Chem. 1994, 373749), synthesis was performed commencing from the last but one residue of Cys(Acm), attached to the polymer by the ester group. After assembly, peptide was oxidized till disulfide on the polymer, then the protected [D-Trp(Boc)⁴, Lys(Boc)⁵, Thr(Bu^{t})⁶] was obtained - octreotide by means of peptidyl-polymer aminolysis by threonine surplus. Amynolysis passed very slowly, and the total output of the protected peptide amounted to 14%.

### Invention disclosure

The aim of the declared invention is to obtain another octapeptide being analogous to somatostatin for the receipt of new radiopharmaceutical preparations with the improved properties, providing therapy of neoplasms expressing somatostatin receptors.

In such a manner, the declared invention describes a group of inventions, which includes octapeptide being octreotide derivative, radiopharmaceutical preparation (RPP) on its basis and the method of radiopharmaceutical means application for the receipt of the medical (pharmaceutical) agents for the radionuclide therapy of neoplasms expressing somatostatin receptors. One of the inventions from the declared group is octapeptide of the general claim (I)

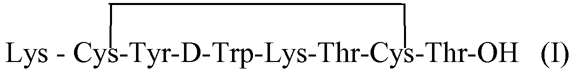

Cyclic octapeptide presented in the invention, differs from the known peptide analogues of octreotide of the general claim D - Phe¹ - Tyr³ (octreotide) is such a way that D - Phe¹ in it is replaced by Lys¹.

Cyclic octapeptide of the claim 1 contains terminal amino acid lysine (Lys), which is able to covalently attach chelating group and has the following structure (Ia) and (Ib) 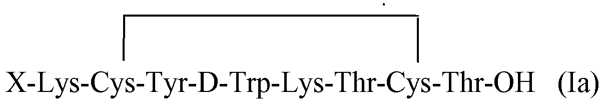 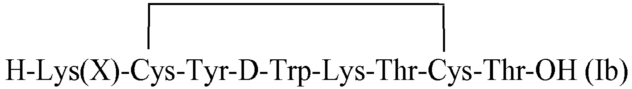 X = chelating agent

Therefore, the declared cyclic octapeptide has a structure containing the above-specified sequence of amino-acids, contains N-terminal amino acid lysine (Lys), capable of covalent attachment of the chelating group, for example DOTA, and is intended for the receipt of radiopharmaceutical preparations with radionuclides ¹¹¹In, ⁹⁰Y, ¹⁷⁷Lu.

The invention also related to the radiopharmaceutical means in the form of compound complex of the claim (I) with radionuclides ¹¹¹In, ⁹⁰Y, ¹⁷⁷Lu. This allows to apply RPP as a therapeutic radiopharmaceutical means for the radionuclide therapy of neoplasms expressing somatostatin receptors. The invention also refers to the method of application of the obtained radiopharmaceutical means application on the basis of octapeptide complex of the claim (I) with radionuclides ¹¹¹In, ⁹⁰Y, ¹⁷⁷Lu, for preparation of the medical (pharmaceutical) agents for the radionuclide therapy of neoplasms expressing somatostatin receptors or their metastases with the help of such radiopharmaceutical preparations.

Along with that, upon radiopharmaceutical preparation obtaining under the invention in the form of octapeptide complex of the claim (I) with radionuclides ¹¹¹In, ⁹⁰Y, ¹⁷⁷Lu, various chelating agents are applied. Chelating agents known in this art are applied and chelators.

In this art related to obtaining of radiopharmaceutical and radiodiagnostical preparations on the basis of peptide carriers with the targeted delivery, the chelating agents can be represented by DOTA (1, 4, 7,10- tetraazacyclododecanetetraacetic acid, as well as other chelating agents known in this art, such as polyaminopolyacid chelating agents NOTA, Oxo, PCTA and other, known and widely used for the radiopharmaceutical preparations (for example, see RU 233557, RU 2010146497, RU 2006105644, US 4647447, US 5362475, EP 230893, WO 95/24225 etc.) and the articles:
- Bioconjug Chem. 2011 Aug 17;22(8) : 1650-62. Epub 2011 Jul 26. Multivalent bifunctional chelator scaffolds for gallium-68 based positron emission tomography imaging probe design: signal amplification via multivalency;
- Bioconjug Chem. 2010 {Fpub ahead of print}
- Evaluation of Bifunctional Chelates for the Development of Gallium - Based Radiopharmaceuticals;
- Etc.

They, in particular, include polyaminopolyacid, bifunctional chelating groups: derivatives of tetraacetic acid (presented in the Latin alphabet):
- NOTA (1,4,7-triazacyclononane-1,4,7-triacetic acid);
- Oxo-(1-oxa-4,7,10-triazacyclododecane-4,7,10- triacetic acid);
- PCTA (3,6,9,15-tetraazabicyclo {9.3.1} pentadeca-1 (15), 11,13-triene - 3,6,9-triacetic acid);
- The above-specified DOTA 1,4,7,10-tetraazacyclododecane-1,4,7-triacetate Therefore, the set objective is achieved by the new octapeptide having structure (I), as the octreotide derivative possessing biological activity and representing interest upon production of the pharmaceutical means applied during diagnostics and treatment, including for creation with its application of various radiopharmaceutical means in the form complexes with different radionuclides.

The suggested invention is illustrated by the corresponding examples not limiting it.

### Synthesis of octapeptides of the general claim 1:

### List of the abbreviations used:

DMF - dimethyl formamide
DIPEA - N, N -diisopropylethylamine
HOBT - N -hydroxybenzothiazole
TBTU - tetrafluoroborate 2-(1H- benzotriazole -1-il)-1,1,3,3-tetrametiluron
TFA - trifluoroacetic acid
Fmoc - fluorenylmethoxycarbonyl
DCC - dicyclohexylcarbodiimide
Trt- trityl
Boc - t-butyloxicarbonyl
But - tretbutyl
P - polymer

Synthesis applied the protected derivates of the Swiss company Bachem. Synthesis of octapeptide analogues of the claim (I) was performed by the solid-phase method.

### Example 1. Synthesis of Fmoc-heptapeptidyl-polymer:

### Cys(Trt)-Tyr-D-Trp-Lys-Thr-Cys(Trt)-Thr-P

Note: symbol «P» means polymer (matrix), on which Fmoc-heptapeptidyl-polymer is grown and which is afterwards deparated and removed, in particular Wang polymer of Bachem company.

Synthesis of peptidyl-polymer - predecessor of octapeptides with the complex-forming group DOTA is performed by the solid-phase method starting from 6.0 g of Fmoc-Thr(But)-Wang ploymer of Bachem company with the content of the initial amino acid of 0.61 mmol/g. Condensation reactions were carried out by the dicyclohexylcarbodiimide method in the presence of N-hydroxy-benzotriazole (DCC/HOBT) in DMF, with the use of double derivative surpluses: Fmoc-Cys(Trt)-OH, Fmoc-Thr(But)-OH, Fmoc-Lys(Boc)-OH, Fmoc-D-Trp-OH, Fmoc-Tyr(But)-OH. For amino acid deblocking, 20% piperidine in dimethyl formamide was used. All operations were performed in compliance with the following protocol (table 1):

**Table 1. Synthesis protocol**

| Item No. | Operation | Treatments number (times) | Treatment duration (min) |
|---|---|---|---|
| 1 | Activation of 0.0074 mmol of Fmoc amino acid in the presence of 0.0074 mmol DCC and 0.0074 mmol HOBT in 30 ml DMF | 1 | 20 |
| 2 | Flushin of peptidyl-polymer 50 ml DMF before deblocking | 3 | 1 |
| 3 | Deblocking of 30 ml of 20% piperidine in DMF | 1 | 5 |
| | | 1 | 15 |
| 4 | Flushing of 50 ml DMF | 5 | 1 |
| 5 | Condensation | 1 | 120 |
| 6 | Flushing of 50 ml DMF | 3 | 1 |

Upon synthesis completion, peptidyl-polymer was filtered, flushed with DMF (3 × 80 ml), dichloromethane (5 × 80 ml) and air-dried. The obtained 10.36 g of Fmoc-heptapeptidyl-polymer were applied at the further stages.

**Example 2. Synthesis of** 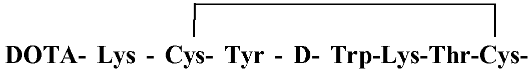 **Thr-OH (I), meeting general claim of octapeptide of the claim (I).**

**Compund (I)** was obtained by means of increment of the peptide chain at the last stage under protocol provided in the table 1, with the use of the last stage of synthesis of Fmoc-Lys(Boc)-OH. 1.0 g (0.5 mmol) of the protected octapeptidyl-polymer was deblocked by 10ml of 20% piperidine DMF and flushed with DMF, as described in the clause 3 and 4 of the table 1.

Peptidyl-polymer was suspended in 30ml DMF, and then 0.47 g (1.0 mmol) of a corresponding lysine derivative was added, as well as 0.15 g (1.0 mmol) of HOBT, 0.32 g (1.0 mmol) of TBTU and 0.5 ml of DIPEA. Reaction mix was stirred for 2 hours under room temperature, and the solvent was removed by filtration, then Fmoc-peptidyl-polymer was flushed, deblocked and flushed again in compliance with the clauses 3 and 4 of the table 1. DOTA(But)₃OH was added with the use of the same reagents as in the case with lysine derivatives. Peptidyl-polymer was filtered, flushed with DMF (3 × 30 ml), dichloromethane (5 × 30 ml) and air-dried. 1.1 g of peptidyl-polymer was suspended in 10.0 ml of the mixture of TFA - thioanisole - tri-izobutylsilane - ethane dithiol - water (8.5 : 0.5 : 0.5 : 0.25 : 0.25), then stirred for 4 hours under room temperature. Resin was filtered, flushed with TFA (2 times per 0.5 ml). Then 50 ml of diethyl ether was added to the filtrate, the precipitation was filtered, flushed on filter with ether (5 ×15 ml), ethyl acetate (2 ×15 ml). Raw product was dissolved in 0.5 ml of water, and then aqueous ammonia was added till pH 9, left overnight under slight stirring under room temperature. 1 ml of 5% H₂O₂solution was added to the reaction mix. Completeness of disulfide link formation was checked with the help of Ellman's reagent. Reaction mixture was boiled down with preliminary adding of 1 ml of vinegar acid (pH 4-5). The product was purified by HPLC method on Diasorb-130 (25×250 mm), elution in the gradient from 0 to 20% during 10 min and from 20% to 60% during 40 minutes of the buffer B in the buffer A (A - 0.1% TFA and B - 80% acetonitrile A), flow rate - 12 ml/min, detection - under 226 nm. Fractions corresponding to the target substance were combine and lyophilized. Compound I output is 0.108 g, which is equal to 15% per initial amino acid, m/z 1416.7 (calculated 1416.0), Rₜ = 13.32 min (analytical HPLC on the chromatograph Gilson, France, column Gromasil C18, 4.6 × 250 mm, gradient of the buffer B content in the buffer A is from 20% to 80% during 30 min, where A - 0.1% TFA and B - 80% acetonitrile in A, flow rate - 1 ml/min).

**Example 3. Synthesis of** 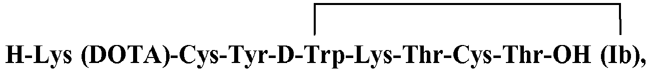 **corresponding to the general oactapeptide claim of the claim (I).**

Compound Ia was obtained similarly to I with the difference that at the last stage of the solid-phase synthesis Boc-Lys(Fmoc)-OH was used. Output (Ib) - 0.099 g, which corresponds to 13% per initial amino acid, m/z 1416.7 (calculated - 1416.0), Rₜ = 13.37 min in the conditions similar to those for the compound (I).

Example of radiopharmaceutical means obtaining in the form of compounds complex of the claim (Ia) and (Ib) with radionuclides ¹¹¹In, ⁹⁰Y, ¹⁷⁷Lu is presented, through the example with radionuclide ¹⁷⁷Lu.

Further examples 4-6 illustrate radiopharmaceutical means (preparation) obtaining under invention and its property.

### Example 4. RPP obtaining on the basis of DOTA-conjugated peptides (Ia) and (Ib), marked by radionuclides ¹¹¹In, ⁹⁰Y, ¹⁷⁷Lu, through the example of ¹⁷⁷Lu.

This methodology describes performance of reaction of marking by radionuclide ¹⁷⁷Lu of DOTA-conjugated peptides - analogues of ocreotide (I) and (Ia), obtained in the examples 2 and 3.

The initial peptide (in the form of a dry substance) is dissolved in DI water (18 mohm) with the concentration of 1 mg/ml. The obtained peptide solution is filled by automatic measurer per 50 mcl in polypropylene test tubes of the type Eppendorf Safe-Lock Tubes (1,5 ml).

Divided peptide solution is kept in the freezing chamber under temperature not above - 18 °C. Prior to marking reaction performance, peptide solution is unfrozen under room temperature within 1-2 minutes. Acetate buffer 0,4 M is added by the measurer to the unfrozen peptide in the same test tube (so that acidity (pH) of the reaction medium shall amount to 4,0-5,0) and transferred beyond the lead protection,where solution ¹⁷⁷Lu (in 0,05 N HCl) is added in the test tube, with the activity of 5-100 mC. Reaction mixture is incubated under 80°C within 20 minutes. Upon 20 minutes expiry, marking reaction is deemed to be completed.

### Example 5. Determination of the radiochemical purity of DOTA-conjugated petides (Ia) and (Ib), marked with radionuclides ¹¹¹In, ⁹⁰Y, ¹⁷⁷Lu, through the example of ¹⁷⁷Lu.

Radiochemical purity of the obtained product (RCP) was determined by the high-performance liquid chromatography (HPLC).

HPLC (UV-detector, 220 nm; eadiodetector Radiomatic Flo-ONE\Beta, Packard) was performed with the use of Acclaim column; under eluent flow rate of 1 ml/min; for the gradient elution the following eluents were used: A - acetonitrile (ACN), B: 0.1% fluoro-acetic acid (TFA).

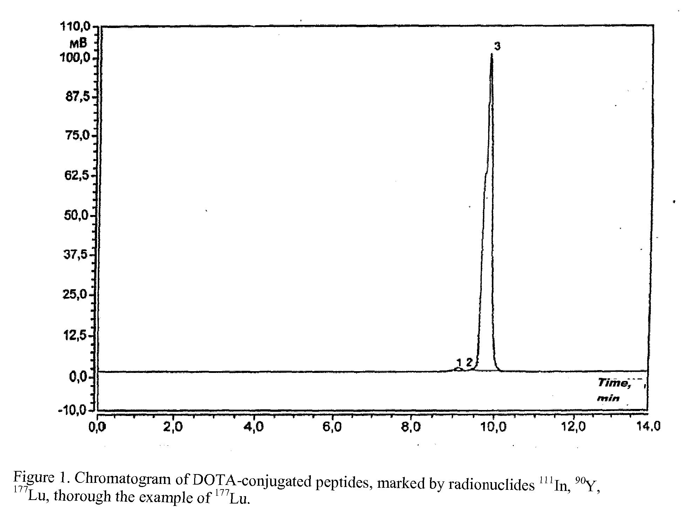

Radiochemical purity amounted to over 98%. Chromatogram is presented in the figure 1.

### Example 6. Study of RPP accumulation on the basis of compounds (Ia) and (Ib), marked with radionuclides ¹¹¹In, ⁹⁰Y, ¹⁷⁷Lu, through the example of ¹¹¹In, among animals with the tangled tumor (melanoma), expressing somatostatin receptors.

Experiments ***in vivo*** were performed applicably to the laboratory female mice (hybrids F1 CBAxC57B1 with the weight of 18 - 20 g). Animals were received from the nursery of the Scientific Center of Biomedical Technologies of the Russian Academy of Medical Sciences "Andreevka". Mice's pigmented melanoma B16 was tangled. In aseptic conditions the suspension of melanoma B16 cells were administered subcutaneously. Tumor growth was observed visually. The animals were included into the experiment in 9-12 after implantation, upon tumor size growth up to 8 - 12 mm in diameter.

During the experiments the animals were kept in the standard conditions (special room, recommended diet, free access to drinking water, natural lighting).

Solutions of the compounds (I) and (Ia), marked with ¹¹¹In, were administered into the tail vein, in 20 and 60 minutes the animals were decapitated, their blood samples were taken, as well as the samples of the muscle and tumor tissues, and the main organs and tissues: liver, kidneys, full urinary bladder, blood, tumor. Urinary bladder was filled by means of ligature application to the external urethral orifice. Radioactivity in the selected organs and tissues was measured by direct

radiometry method. At least 3 animals we used for each temporary point. The obtained data are presented in the table 2.

**Table 2. Activity distribution in the organism of mice C57B1 with the tangled melanoma B16 (% of the administered dose).**

| Organ/tissue | 20 minutes | 60 minutes |
|---|---|---|
| | % of the administered dose | |
| Blood | 3.5 | 1.6 |
| Liver | 2.9 | 3.4 |
| Kidneys | 4.4 | 5.1 |
| Urinary bladder | 53.3 | 73.2 |
| Muscles, %/g | 1.7 | 1.2 |
| Tumor, %/g | 5.2 | 2.6 |
| Tumor/muscle | 3.1 | 2.2 |
| Timor/blood | 1.5 | 1.6 |

As it is obvious from the presented data, the obtained compound demonstrates affinity to the tumor melanoma B 16 in vivo. According to the obtained data, the preparation is almost completely excreted through the kidneys, moderately accumulates in the liver and has a quite high ratio "tumor/muscle and tumor/blood".

Aggregate results of marking and biological behavior in vivo allow to consider application of the compounds (I) and (Ia) marked by ¹¹¹In, ¹⁷⁷Lu, ⁹⁰Y, and other radionuclides reasonable for the radionuclide therapy of neoplasms (tumors) expressing somatostatin receptors.

The table below explains figure 1.

| Item No. | Peak | Time, min | Area, mV*min | Height, mV | Area, % | Height % | Score |
|---|---|---|---|---|---|---|---|
| 1 | A | 9.003 | 0.029 | 0.271 | 0.14 | 0.27 | - |
| 2 | B | 9.131 | 0.131 | 0.827 | 0.63 | 0.82 | - |
| 3 | C | 9.816 | 20.804 | 99.197 | 99.24 | 98.91 | - |
| Total | | | 20.964 | 100.295 | 100.00 | 100.00 | |

## Claims

1. Cyclic octapeptide having the following structure (I) and containing the following amino acid sequence:

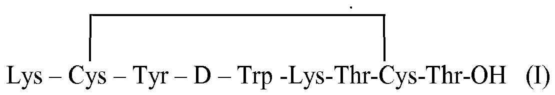

2. Cyclic octapeptide under claim 1 is distinguished by content of amino acid lysine (Lys), capable of covalent attachment of the chelating group and having the following structure (Ia) and (Ib) 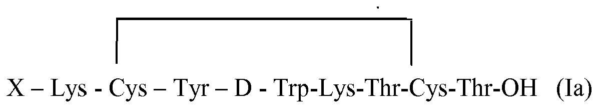 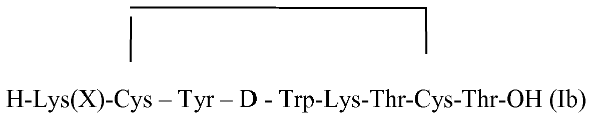 X = chelating agent

3. Radiopharmaceutical means including octapeptide complex under claim 1 or 2 with radionuclides ¹¹¹In, ⁹⁰Y, ¹⁷⁷Lu, formed with the use if the complex-forming agent.

4. Method of pharmaceutical means application under claim 3 for obtaining of the medical (pharmaceutical) means for radionuclide therapy of neoplasms expressing somatostatin receptors.
